# EUROPEAN PATENT APPLICATION

(11) **EP 4 847 013 A1**
(43) Date of publication of application: **02.12.2026**
(21) Application number: 25744721.9
(22) Date of filing: 24.01.2025
(51) Int. Cl.: A61K 9/28, A61K 31/4725, A61K 31/4741, A61K 47/38, A61K 47/02, A61P 15/00, A61P 35/00, A61P 35/02

(54) **PHARMACEUTICAL COMPOSITION COMPRISING SELECTIVE ESTROGEN RECEPTOR DEGRADER, AND PREPARATION METHOD THEREFOR**

(30) Priority: 25.01.2024 CN 202410105317
(71) Applicant: Shandong Suncadia Medicine Co., Ltd., Jinan, Shandong 250104 (CN); Jiangsu Hengrui Pharmaceuticals Co., Ltd., Lianyungang, Jiangsu 222047 (CN)
(72) Inventor: SU, Xun, Jinan, Shandong 250104 (CN); HUANG, Yinghao, Jinan, Shandong 250104 (CN); WANG, Ya, Jinan, Shandong 250104 (CN); GUO, Chuanglong, Jinan, Shandong 250104 (CN); ZHU, Ning, Jinan, Shandong 250104 (CN)
(74) Representative: Potter Clarkson
(86) International application number: PCT/CN2025/074644
(87) International publication number: WO 2025/157258

(57) **Abstract**

The present disclosure relates to a pharmaceutical composition comprising a selective estrogen receptor degrader, and a preparation method therefor. Specifically, the present disclosure provides a composition, comprising a compound represented by formula (I) or a pharmaceutically acceptable salt thereof and a filler, wherein the filler comprises microcrystalline cellulose and anhydrous dibasic calcium phosphate. The composition has good dissolution and stability.

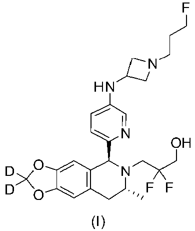

## Description

### TECHNICAL FIELD

The present disclosure pertains to the field of pharmaceutical formulations, and particularly relates to a pharmaceutical composition comprising a selective estrogen receptor degrader (SERD) and a preparation method therefor. Specifically, the present disclosure relates to a composition comprising 2,2-difluoro-3-((5S,7R)-5-(5-((1-(3-fluoropropyl)azetidin-3-yl)amino)pyridin-2-yl)-7-m ethyl-7,8-dihydro-[1,3]dioxolo[4,5-g]isoquinolin-6(5H)-yl-2,2-d₂)propan-1-ol or a pharmaceutically acceptable salt thereof and a filler, wherein the filler comprises microcrystalline cellulose and anhydrous dibasic calcium phosphate.

### BACKGROUND

Fulvestrant in the CSCO Guidelines for Breast Cancer Diagnosis and Treatment (2020 Edition) has been upgraded to a class I recommendation for patients with hormone receptor-positive advanced breast cancer who have not previously received endocrine therapy, and fulvestrant + CDK4/6 inhibitor has been added as a class II recommendation. In addition, fulvestrant combined with the CDK4/6 inhibitor has become a class I recommendation for patients who have failed nonsteroidal AI (NSAI) therapy and steroidal AI (SAI) therapy. As the only approved SERD at present, fulvestrant is approved as an injection, which is inconvenient for patients to take. Therefore, the search for a new and more effective SERD not only has great market value, but also brings medication selectivity and convenience for breast cancer patients. SAI439859 from Sanofi is currently in clinical phase III as a new generation of orally available SERD inhibitor; in addition, GDC-9545 from Roche, AZD-9833 from AstraZeneca, and RAD1901 from Radius have all entered phase III.

Disclosed in WO2021139756A1 is a selective estrogen receptor degrader (SERD), which is a compound represented by formula (I) with the name of 2,2-difluoro-3-((5S,7R)-5-(5-((1-(3-fluoropropyl)azetidin-3-yl)amino)pyridin-2-yl)-7-m ethyl-7,8-dihydro-[1,3]dioxolo[4,5-g]isoquinolin-6(5H)-yl-2,2-d₂)propan-1-ol, and the compound shows a significant degradation effect on ERα,

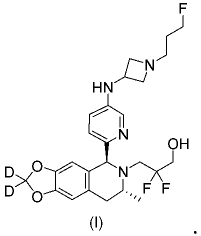

To meet the medication needs of patients, the compound of formula (I) 2,2-difluoro-3-((5S,7R)-5-(5-((1-(3-fluoropropyl)azetidin-3-yl)amino)pyridin-2-yl)-7-m ethyl-7,8-dihydro-[1,3]dioxolo[4,5-g]isoquinolin-6(5H)-yl-2,2-d₂)propan-1-ol described above or a pharmaceutically acceptable salt thereof needs to be prepared into a suitable formulation, which needs to have good dissolution properties and formulation stability in terms of solid formulations. In terms of tablets, the tablets should have an intact and smooth appearance, uniform color, and suitable hardness and abrasion resistance.

### SUMMARY

The present disclosure provides a pharmaceutical composition comprising a compound represented by formula (I) or a pharmaceutically acceptable salt thereof as an active ingredient and a filler, wherein the filler comprises microcrystalline cellulose and anhydrous dibasic calcium phosphate,

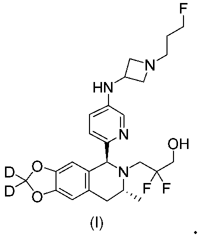

In some embodiments, the weight ratio of microcrystalline cellulose to anhydrous dibasic calcium phosphate is 1:2 to 2:1.

In some specific embodiments, the weight ratio of microcrystalline cellulose to anhydrous dibasic calcium phosphate is 1:2 to 1:1.

In some embodiments, the content of the active ingredient is 0.1%-40% based on the total weight of the pharmaceutical composition.

In some embodiments, the content of the active ingredient is 1%-35% based on the total weight of the pharmaceutical composition, and optional contents include 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, or a numerical value between any two point values.

In some embodiments, the content of the compound represented by formula (I) in the unit dosage form of the pharmaceutical composition is selected from the group consisting of 1 mg to 500 mg (based on the free base form); specifically, the content may be 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, 200 mg, 205 mg, 210 mg, 215 mg, 220 mg, 225 mg, 230 mg, 235 mg, 240 mg, 245 mg, 250 mg, 255 mg, 260 mg, 265 mg, 270 mg, 275 mg, 280 mg, 285 mg, 290 mg, 295 mg, 300 mg, 305 mg, 310 mg, 315 mg, 320 mg, 325 mg, 330 mg, 335 mg, 340 mg, 345 mg, 350 mg, 355 mg, 360 mg, 365 mg, 370 mg, 375 mg, 380 mg, 385 mg, 390 mg, 395 mg, 400 mg, 405 mg, 410 mg, 415 mg, 420 mg, 425 mg, 430 mg, 435 mg, 440 mg, 445 mg, 450 mg, 455 mg, 460 mg, 465 mg, 470 mg, 475 mg, 480 mg, 485 mg, 490 mg, 495 mg, or 500 mg.

In some embodiments, the content of the compound represented by formula (I) in the unit dosage form of the pharmaceutical composition is selected from the group consisting of 25 mg to 200 mg (based on the free base form).

In some embodiments, the content of the compound represented by formula (I) in the unit dosage form of the pharmaceutical composition is selected from the group consisting of 50 mg, 75 mg, 100 mg, 150 mg, and 200 mg (based on the free base form). In some specific embodiments, the content of the compound represented by formula (I) in the unit dosage form of the pharmaceutical composition is 50 mg (based on the free base form).

In some specific embodiments, the content of the compound represented by formula (I) in the unit dosage form of the pharmaceutical composition is 75 mg (based on the free base form).

In some specific embodiments, the content of the compound represented by formula (I) in the unit dosage form of the pharmaceutical composition is 100 mg (based on free base form).

In some specific embodiments, the content of the compound represented by formula (I) in the unit dosage form of the pharmaceutical composition is 150 mg (based on free base form).

In some specific embodiments, the content of the compound represented by formula (I) in the unit dosage form of the pharmaceutical composition is 200 mg (based on the free base form).

In some embodiments, the content of the filler is 20%-95% based on the total weight of the pharmaceutical composition.

In some embodiments, the content of the filler is 50%-95% based on the total weight of the pharmaceutical composition, and optional contents include 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, or a numerical value between any two point values.

In some embodiments, the content of microcrystalline cellulose is 10%-50% based on the total weight of the pharmaceutical composition.

In some embodiments, the content of microcrystalline cellulose is 15%-45% based on the total weight of the pharmaceutical composition, and optional contents include 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, or a numerical value between any two point values.

In some embodiments, the content of anhydrous dibasic calcium phosphate is 10%-50% based on the total weight of the pharmaceutical composition.

In some embodiments, the content of anhydrous dibasic calcium phosphate is 15%-45% based on the total weight of the pharmaceutical composition, and optional contents include 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, or a numerical value between any two point values.

In some embodiments, the pharmaceutical composition further comprises one or more of a disintegrant, a binder, a glidant, a lubricant, and a coating agent.

In some embodiments, the content of the disintegrant is 1%-5% based on the total weight of the pharmaceutical composition.

In some embodiments, the content of the disintegrant is 1.5%-5% based on the total weight of the pharmaceutical composition, and optional contents include 1.5%, 1.6%, 1.7%, 1.8%, 1.9%, 2.0%, 2.2%, 2.4%, 2.6%, 2.8%, 3.0%, 3.2%, 3.4%, 3.6%, 3.8%, 4.0%, 4.2%, 4.4%, 4.6%, 4.8%, 5.0%, or a numerical value between any two point values.

In some specific embodiments, the disintegrant is croscarmellose sodium.

In some embodiments, the content of the binder is 0.1%-10% based on the total weight of the pharmaceutical composition.

In some embodiments, the content of the binder is 0.2%-5% based on the total weight of the pharmaceutical composition, and optional contents include 0.2%, 0.3%, 0.4%, 0.5%, 0.6%, 0.7%, 0.8%, 0.9%, 1.0%, 1.2%, 1.4%, 1.6%, 1.8%, 2.0%, 2.2%, 2.4%, 2.6%, 2.8%, 3.0%, 3.2%, 3.4%, 3.6%, 3.8%, 4.0%, 4.2%, 4.4%, 4.6%, 4.8%, 5.0%, or a numerical value between any two point values.

In some specific embodiments, the binder is hydroxypropyl cellulose.

In some embodiments, the content of the glidant is 0.1%-10% based on the total weight of the pharmaceutical composition.

In some embodiments, the content of the glidant is 0.2%-5% based on the total weight of the pharmaceutical composition, and optional contents include 0.2%, 0.3%, 0.4%, 0.5%, 0.6%, 0.7%, 0.8%, 0.9%, 1.0%, 1.2%, 1.4%, 1.6%, 1.8%, 2.0%, 2.2%, 2.4%, 2.6%, 2.8%, 3.0%, 3.2%, 3.4%, 3.6%, 3.8%, 4.0%, 4.2%, 4.4%, 4.6%, 4.8%, 5.0%, or a numerical value between any two point values.

In some specific embodiments, the glidant is colloidal silicon dioxide.

In some embodiments, the content of the lubricant is 0.1%-10% based on the total weight of the pharmaceutical composition.

In some embodiments, the content of the lubricant is 0.2%-5% based on the total weight of the pharmaceutical composition, and optional contents include 0.2%, 0.3%, 0.4%, 0.5%, 0.6%, 0.7%, 0.8%, 0.9%, 1.0%, 1.2%, 1.4%, 1.6%, 1.8%, 2.0%, 2.2%, 2.4%, 2.6%, 2.8%, 3.0%, 3.2%, 3.4%, 3.6%, 3.8%, 4.0%, 4.2%, 4.4%, 4.6%, 4.8%, 5.0%, or a numerical value between any two point values.

In some specific embodiments, the lubricant is magnesium stearate.

In some embodiments, the coating agent is a film coating premix (gastric soluble).

In some embodiments, the coating weight gain is 2%-4% based on the total weight of the pharmaceutical composition.

In some embodiments, the coating weight gain is 2.5%-3.5% based on the total weight of the pharmaceutical composition, and optional coating weight gains include 2.5%, 2.6%, 2.7%, 2.8%, 2.9%, 3.0%, 3.1%, 3.2%, 3.3%, 3.4%, 3.5%, or a numerical value between any two point values.

In some embodiments, the pharmaceutically acceptable salt as the active ingredient is a triphosphate.

In some specific embodiments, the pharmaceutical composition comprises, based on the total weight of the pharmaceutical composition,
1%-35% of an active ingredient, the active ingredient being the compound represented by formula (I) or the pharmaceutically acceptable salt thereof, 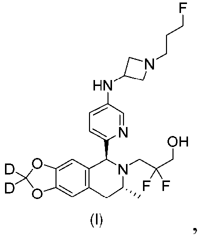
50%-95% of a filler, the filler being microcrystalline cellulose and anhydrous dibasic calcium phosphate,
1.5%-5% of a disintegrant, the disintegrant being croscarmellose sodium,
0.2%-5% of a binder, the binder being hydroxypropyl cellulose,
0.2%-5% of a glidant, the glidant being colloidal silicon dioxide,
0.2%-5% of a lubricant, the lubricant being magnesium stearate,
wherein the coating agent is a film coating premix (gastric soluble), and the coating weight gain is 2.5%-3.5% based on the total weight of the pharmaceutical composition,
the content of the compound represented by formula (I) in the unit dosage form is selected from the group consisting of 50 mg, 75 mg, 100 mg, 150 mg, and 200 mg (based on the free base form).

In some embodiments, the weight ratio of microcrystalline cellulose to anhydrous dibasic calcium phosphate is 1:2 to 2:1.

In some specific embodiments, the weight ratio of microcrystalline cellulose to anhydrous dibasic calcium phosphate is 1:2 to 1:1.

In some embodiments, the composition is present in the form of a tablet.

In some embodiments, the composition is subjected to dissolution rate determination according to the Dissolution and Drug Release Test Method 2 (Paddle) of General Chapter 0931 in Chinese Pharmacopoeia (Volume IV, 2020 Edition), and the active ingredient has a cumulative dissolution rate of ≥ 85% at 30 min, including ≥ 85%, 86%, 87%, 88%, 89%, 90%, 91%, 92%, 93%, 94%, 95%, 96%, 97%, 98%, 99%, or 100%.

In some embodiments, the active ingredient has a cumulative dissolution rate of ≥ 90% at 45 min, including ≥ 90%, 91%, 92%, 93%, 94%, 95%, 96%, 97%, 98%, 99%, or 100%.

In some specific embodiments, the active ingredient has a cumulative dissolution rate of ≥ 94% at 45 min, including ≥ 94%, 95%, 96%, 97%, 98%, 99%, or 100%.

The present disclosure further provides a method for preparing the pharmaceutical composition described in the present disclosure, comprising:
1) mixing the compound of formula (I) or the pharmaceutically acceptable salt thereof with the filler and optionally at least one pharmaceutical auxiliary material selected from the group consisting of the disintegrant, the binder, the glidant, and the lubricant; and
2) performing dry granulation on the mixture obtained in step 1), followed by compression into a tablet.

In some embodiments, the method further comprises a step of coating the tablet obtained in step 2).

In some specific embodiments, the step of coating the tablet obtained in step 2) comprises coating the tablet obtained in step 2) with a film coating premix (gastric soluble) and water.

The present disclosure further provides use of the pharmaceutical composition described in the present disclosure in the preparation of a selective estrogen receptor degrader (SERD).

The present disclosure further provides use of the pharmaceutical composition described in the present disclosure in the manufacture of a medicament for preventing and/or treating cancer, wherein the cancer is selected from the group consisting of breast cancer, endometrial cancer, cervical cancer, skin cancer, prostate cancer, ovarian cancer, fallopian tube tumor, ovarian tumor, hemophilia, and leukemia.

In some embodiments, the cancer is selected from the group consisting of breast cancer, ovarian cancer, endometrial cancer, prostate cancer, and uterine cancer.

In some specific embodiments, the cancer is breast cancer.

The present disclosure further provides a method for treating cancer, the method comprising administering to a patient in need thereof a therapeutically effective amount of the pharmaceutical composition described in the present disclosure, wherein the cancer is selected from the group consisting of breast cancer, endometrial cancer, cervical cancer, skin cancer, prostate cancer, ovarian cancer, fallopian tube tumor, ovarian tumor, hemophilia, and leukemia.

In some embodiments, the cancer is selected from the group consisting of breast cancer, ovarian cancer, endometrial cancer, prostate cancer, and uterine cancer.

In some specific embodiments, the cancer is breast cancer.

The present disclosure further provides the pharmaceutical composition described in the present disclosure for use as a medicament for treating cancer, wherein the cancer is selected from the group consisting of breast cancer, endometrial cancer, cervical cancer, skin cancer, prostate cancer, ovarian cancer, fallopian tube tumor, ovarian tumor, hemophilia, and leukemia.

In some embodiments, the cancer is selected from the group consisting of breast cancer, ovarian cancer, endometrial cancer, prostate cancer, and uterine cancer.

In some specific embodiments, the cancer is breast cancer.

### Terms and Definitions

In order to facilitate the understanding of the present disclosure, certain technical and scientific terms are specifically defined below. Unless otherwise specifically defined in the present disclosure, all other technical and scientific terms used in the present disclosure have the meanings generally understood by those of ordinary skill in the art to which the present disclosure pertains.

"Pharmaceutical composition" refers to a mixture containing one or more of the active ingredients or the physiologically/pharmaceutically acceptable salts or pro-drugs thereof described herein, and other chemical components, as well as other components, for example, physiologically/pharmaceutically acceptable carriers and excipients. The pharmaceutical composition is intended to promote administration to an organism and facilitate the absorption of the active ingredient so that it can exert its biological activity. The pharmaceutically acceptable salts of the compound described in the present disclosure may be selected from the group consisting of inorganic salts and organic salts.

"Based on the total weight of the pharmaceutical composition" described in the present disclosure refers to the numerical range of the amount of the active ingredient or other pharmaceutical auxiliary materials calculated on the basis of the weight of the tablet core without the coating agent.

"Mixed" in the present disclosure means that the order of addition of the components is not limited. For example, when A is mixed into B, it can mean either A is added to B or B is added to A; when A and B are mixed, it can mean either A is added to B and mixed or B is added to A and mixed.

"Treat", "treating", or "treatment" refers to administering a therapeutic agent, such as a therapeutic agent comprising any one of the fusion proteins or the insulin analogs of the present disclosure, to a subject who has had, is suspected of having, or is predisposed to having one or more types of diabetes or hyperglycemia-related diseases or symptoms thereof on which the therapeutic agent is known to have a therapeutic effect. Generally, the therapeutic agent is administered in an amount effective to alleviate one or more disease symptoms in the subject or population being treated. This is achieved by preventing or delaying the onset of the symptoms or complications, alleviating the symptoms or complications, or eliminating the disease, condition, or disorder to any clinically measurable degree. The amount of therapeutic agent effective in alleviating any specific disease symptom (also referred to as the "therapeutically effective amount") may vary depending on factors such as the disease state, age, and body weight of the subject, and the ability of the drug to produce a desired therapeutic effect in the subject. Whether a disease symptom has been palliated can be evaluated by any clinical test methods commonly used by doctors or other health care professionals to evaluate the severity or progression of the symptom. Although embodiments of the present disclosure (e.g., treatment methods or articles of manufacture) may be ineffective in alleviating a disease symptom of interest in a certain subject, they shall palliate the disease symptom of interest in a statistically significant number of subjects as determined by any statistical testing method known in the art, such as a Student's t-test, a Chi-square test, a U-test by Mann and Whitney, a Kruskal-Wallis test (H-test), a Jonckheere-Terpstra test, and a Wilcoxon test. The patient to be treated is a mammal, preferably a human.

"Prevent", "preventing", or "prevention" refers to reducing the risk or incidence of one or more conditions, symptoms, complications or disorders, or eliminating or slowing the progression of one or more conditions, symptoms, complications or disorders.

"Optional" or "optionally" means that the event or circumstance subsequently described may, but does not necessarily, occur. This description includes the instance where the event or circumstance occurs or does not occur.

"Subject" or "patient" means a mammal, particularly a primate, and especially a human. Unless the context clearly requires otherwise, throughout the description and the claims, the words "comprise", "have", "include", etc., are to be understood in an inclusive sense as opposed to an exclusive or exhaustive sense, that is to say, in the sense of "including but not limited to".

### BRIEF DESCRIPTION OF THE DRAWING

FIG. 1 shows the appearance of the coated tablets prepared according to formulation 1 of Example 1.
FIG. 2 shows the appearance of the coated tablets prepared according to formulation 2 of Example 1.
FIG. 3 shows the appearance of the coated tablets prepared according to formulation 3 of Example 1.
FIG. 4 shows a comparison of the appearance of the coated tablets prepared according to formulations 1-3 of Example 1, where the coated tablets prepared according to formulations 1, 2, and 3 are arranged from right to left.

### DETAILED DESCRIPTION

The present disclosure is further described with reference to the following examples; however, these examples are not intended to limit the scope of the present disclosure. Experimental procedures without specific conditions indicated in the examples or test examples of the present disclosure are generally conducted under conventional conditions, or under conditions recommended by the manufacturers of the starting materials or commercial products. Reagents without specific sources indicated were commercially available conventional reagents.

### Example 1: Preparation of Tablets

**Table 1. Formulations of pharmaceutical compositions**

| Formulation | Formulation 1 (mg/tablet) | Formulation 2 (mg/tablet) | Formulation 3 (mg/tablet) |
|---|---|---|---|
| Triphosphate of compound of formula (I) | 318.0 | 318.0 | 318.0 |
| Microcrystalline cellulose | 199.0 | 298.5 | 398.0 |
| Anhydrous dibasic calcium phosphate | 398.0 | 298.5 | 199.0 |
| Croscarmellose sodium | 46.0 | 46.0 | 46.0 |
| Hydroxypropyl cellulose | 11.0 | 11.0 | 11.0 |
| Magnesium stearate | 14.0 | 14.0 | 14.0 |
| Colloidal silicon dioxide | 14.0 | 14.0 | 14.0 |
| Total amount | 1000 | 1000 | 1000 |

The materials were weighed out according to the above table. The triphosphate of the compound of formula (I), microcrystalline cellulose, anhydrous dibasic calcium phosphate, hydroxypropyl cellulose, part of croscarmellose sodium, and part of magnesium stearate were mixed, and granules were prepared using a dry granulator. The remaining croscarmellose sodium and colloidal silicon dioxide were added to the granules described above, and then the remaining magnesium stearate was added. The mixture was mixed to give final mixed granules. The final mixed granules were compressed into tablets.

The tablets obtained from the previous compression were coated with a film coating premix (gastric soluble) and water to give coated tablets.

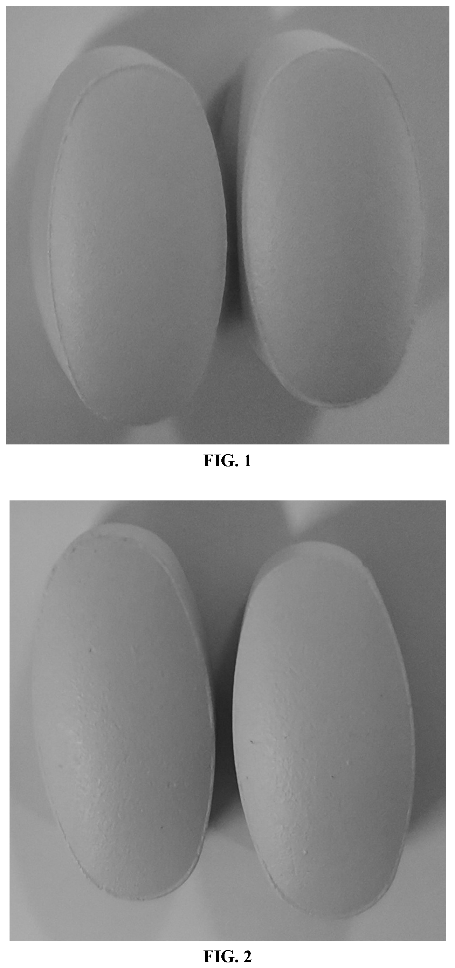

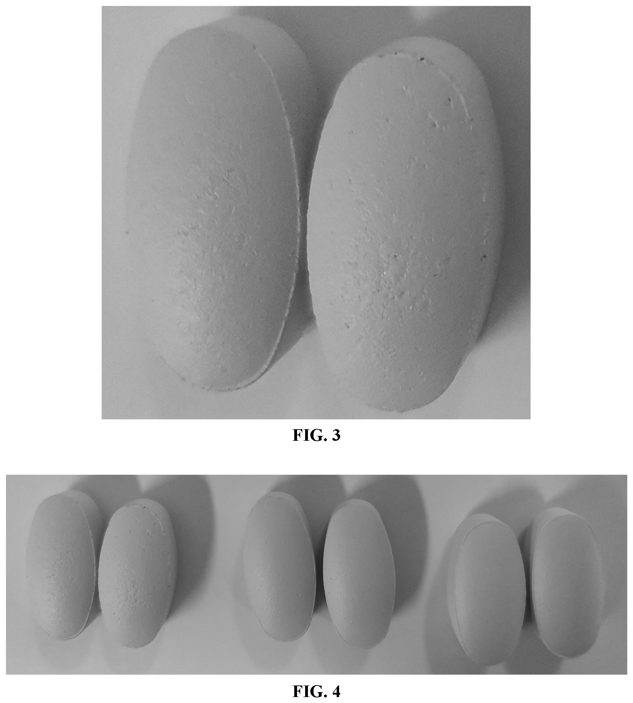

According to the scheme described above, coated tablets of formulation 1, formulation 2, and formulation 3 were prepared, with 300 tablets for each formulation, and their appearances were observed. As shown in FIGs. 1 and 2, in the prepared 300 coated tablets, the coated tablets of formulation 1 and formulation 2 all exhibited smooth surfaces without obvious depressions, and the qualification rates were 100%. As shown in FIGs. 3 and 4, compared with the coated tablets of formulation 1 and formulation 2, in the prepared 300 coated tablets, the coated tablets of formulation 3 all exhibited obvious depressions on the surface, and the qualification rate was 0.

### Example 2: Dissolution Rate Test

According to the Dissolution and Drug Release Test (Method 2 of General Chapter 0931 in Chinese Pharmacopoeia (Volume IV, 2020 Edition)), 900 mL of an acetate buffer at a pH of 4.5 was used as a dissolution medium, and the rotation speed was 75 rpm. At 5 min, 10 min, 15 min, 30 min, 45 min, and 60 min, an appropriate amount of the solution was taken and filtered to give a test sample solution.

Reference solution: An appropriate amount of the triphosphate of the compound of formula (I) was dissolved in the dissolution medium and diluted quantitatively to give a control sample solution.

The test sample solutions and the reference solution were separately injected into a liquid chromatograph, the chromatogram was recorded, the peak area was measured, and the dissolution amount of each tablet at different time points was calculated (see Table 2).

**Table 2. Dissolution rates of different tablets**

| Formulation | Cumulative dissolution (%) | | | | | |
|---|---|---|---|---|---|---|
| | 5 min | 10 min | 15 min | 30 min | 45 min | 60 min |
| Formulation 1 | 19 | 51 | 77 | 92 | 94 | 94 |
| Formulation 2 | 11 | 35 | 57 | 88 | 97 | 93 |
| Formulation 3 | 20 | 43 | 69 | 96 | 97 | 97 |

### Example 3: Accelerated Test

The coated tablets of formulations 1 and 3 were prepared according to the preparation method for tablets in Example 1, placed in high-density polyethylene bottles, and left to stand under conditions of 40 °C and RH 75% for 3 months. The contents of total impurities in the coated tablets of formulations 1 and 3 were determined by HPLC. The test results are shown in Table 3.

**Table 3. Stability of different tablets**

| Formulation | Formulation 1 | | | Formulation 3 | | |
|---|---|---|---|---|---|---|
| Sampling point | 0 days | 1 month | 3 months | 0 days | 1 month | 3 months |
| Total impurity (%) (limit ≤ 1.5%) | 0.06 | 0.05 | 0.05 | 0.25 | 0.40 | 0.54 |

## Claims

1. A pharmaceutical composition, comprising a compound represented by formula (I) or a pharmaceutically acceptable salt thereof as an active ingredient and a filler, wherein the filler comprises microcrystalline cellulose and anhydrous dibasic calcium phosphate, 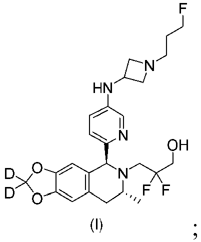
preferably, the weight ratio of microcrystalline cellulose to anhydrous dibasic calcium phosphate is 1:2 to 2:1;
more preferably, the weight ratio of microcrystalline cellulose to anhydrous dibasic calcium phosphate is 1:2 to 1:1.

2. The pharmaceutical composition according to claim 1, wherein the content of the active ingredient is 0.1%-40% based on the total weight of the pharmaceutical composition;
preferably, the content of the active ingredient is 1%-35% based on the total weight of the pharmaceutical composition.

3. The pharmaceutical composition according to claim 1 or 2, wherein the content of the filler is 20%-95% based on the total weight of the pharmaceutical composition;
preferably, the content of the filler is 50%-95% based on the total weight of the pharmaceutical composition.

4. The pharmaceutical composition according to any one of claims 1-3, wherein the content of the compound represented by formula (I) in a unit dosage form is selected from the group consisting of 1 mg to 500 mg (based on the free base form);
preferably, the content of the compound represented by formula (I) in the unit dosage form is selected from the group consisting of 25 mg to 200 mg (based on the free base form);
more preferably, the content of the compound represented by formula (I) in the unit dosage form is selected from the group consisting of 50 mg, 75 mg, 100 mg, 150 mg, and 200 mg (based on the free base form).

5. The pharmaceutical composition according to any one of claims 1-4, wherein the content of microcrystalline cellulose is 10%-50% based on the total weight of the pharmaceutical composition;
preferably, the content of microcrystalline cellulose is 15%-45% based on the total weight of the pharmaceutical composition.

6. The pharmaceutical composition according to any one of claims 1-5, wherein the content of anhydrous dibasic calcium phosphate is 10%-50% based on the total weight of the pharmaceutical composition;
preferably, the content of anhydrous dibasic calcium phosphate is 15%-45% based on the total weight of the pharmaceutical composition.

7. The pharmaceutical composition according to any one of claims 1-6, further comprising one or more of a disintegrant, a binder, a glidant, a lubricant, and a coating agent.

8. The pharmaceutical composition according to claim 7, wherein the content of the disintegrant is 1%-5% based on the total weight of the pharmaceutical composition;
preferably, the content of the disintegrant is 1.5%-5% based on the total weight of the pharmaceutical composition.

9. The pharmaceutical composition according to claim 7 or 8, wherein the disintegrant is croscarmellose sodium.

10. The pharmaceutical composition according to claim 7, wherein the content of the binder is 0.1%-10% based on the total weight of the pharmaceutical composition;
preferably, the content of the binder is 0.2%-5% based on the total weight of the pharmaceutical composition.

11. The pharmaceutical composition according to claim 7 or 10, wherein the binder is hydroxypropyl cellulose.

12. The pharmaceutical composition according to claim 7, wherein the content of the glidant is 0.1%-10% based on the total weight of the pharmaceutical composition;
preferably, the content of the glidant is 0.2%-5% based on the total weight of the pharmaceutical composition.

13. The pharmaceutical composition according to claim 7 or 12, wherein the glidant is colloidal silicon dioxide.

14. The pharmaceutical composition according to claim 7, wherein the content of the lubricant is 0.1%-10% based on the total weight of the pharmaceutical composition;
preferably, the content of the lubricant is 0.2%-5% based on the total weight of the pharmaceutical composition.

15. The pharmaceutical composition according to claim 7 or 14, wherein the lubricant is magnesium stearate.

16. The pharmaceutical composition according to claim 7, wherein the coating agent is a film coating premix (gastric soluble).

17. The pharmaceutical composition according to claim 7 or 16, wherein the coating weight gain is 2%-4% based on the total weight of the pharmaceutical composition; preferably, the coating weight gain is 2.5%-3.5% based on the total weight of the pharmaceutical composition.

18. The pharmaceutical composition according to any one of claims 1-17, wherein the pharmaceutically acceptable salt as the active ingredient is a triphosphate.

19. The pharmaceutical composition according to any one of claims 1-18, comprising, based on the total weight of the pharmaceutical composition,
1%-35% of an active ingredient, the active ingredient being the compound represented by formula (I) or the pharmaceutically acceptable salt thereof, 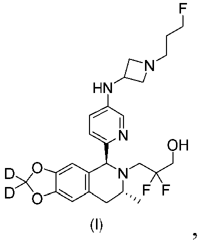
50%-95% of a filler, the filler being microcrystalline cellulose and anhydrous dibasic calcium phosphate,
1.5%-5% of a disintegrant, the disintegrant being croscarmellose sodium,
0.2%-5% of a binder, the binder being hydroxypropyl cellulose,
0.2%-5% of a glidant, the glidant being colloidal silicon dioxide,
0.2%-5% of a lubricant, the lubricant being magnesium stearate,
wherein the coating agent is a film coating premix (gastric soluble), and the coating weight gain is 2.5%-3.5% based on the total weight of the pharmaceutical composition,
the content of the compound of formula (I) in the unit dosage form is selected from the group consisting of 50 mg, 75 mg, 100 mg, 150 mg, and 200 mg (based on the free base form);
preferably, the weight ratio of microcrystalline cellulose to anhydrous dibasic calcium phosphate is 1:2 to 2:1;
more preferably, the weight ratio of microcrystalline cellulose to anhydrous dibasic calcium phosphate is 1:2 to 1:1.

20. The pharmaceutical composition according to any one of claims 1-17, wherein the composition is present in the form of a tablet.

21. The pharmaceutical composition according to any one of claims 1-18, wherein the composition is subjected to dissolution rate determination according to the Dissolution and Drug Release Test Method 2 (Paddle) of General Chapter 0931 in Chinese Pharmacopoeia (Volume IV, 2020 Edition), and the active ingredient has a cumulative dissolution rate of ≥ 85% at 30 min.

22. The pharmaceutical composition according to claim 21, wherein the active ingredient has a cumulative dissolution rate of ≥ 90% at 45 min;
preferably, the active ingredient has a cumulative dissolution rate of ≥ 94% at 45 min.

23. A method for preparing the pharmaceutical composition according to any one of claims 1-22, comprising:
1) mixing the compound of formula (I) or the pharmaceutically acceptable salt thereof with the filler and optionally at least one pharmaceutical auxiliary material selected from the group consisting of the disintegrant, the binder, the glidant, and the lubricant; and
2) performing dry granulation on the mixture obtained in step 1), followed by compression into a tablet;
preferably, further comprising a step of coating the tablet obtained in step 2).
